Europäisches Patentamt

European Patent Office

Office européen des brevets

(19)

(11) Veröffentlichungsnummer: **0 159 540**
**B1**

(12) **EUROPÄISCHE PATENTSCHRIFT**

(45) Veröffentlichungstag der Patentschrift:
26.10.88

(21) Anmeldenummer: 85103272.2

(22) Anmeldetag: 21.03.85

(51) Int. Cl.⁴: **A 61 N 1/05,** A 61 B 5/04

(54) **Chirurgische Elektrode.**

(30) Priorität: 06.04.84 DE 3412950

(43) Veröffentlichungstag der Anmeldung:
30.10.85 Patentblatt 85/44

(45) Bekanntmachung des Hinweises auf die Patenterteilung:
26.10.88 Patentblatt 88/43

(84) Benannte Vertragsstaaten:
AT BE CH DE FR IT LI NL

(56) Entgegenhaltungen:
EP - A - 0 009 732
EP - A - 0 049 780
EP - A - 0 071 495
EP - A - 0 083 674
WO - A - 80/02801
DE - A - 2 516 712
DE - A - 2 929 189
DE - A - 3 043 189
DE - A - 3 046 667
FR - A - 2 483 786
US - A - 3 769 984
US - A - 4 010 756
US - A - 4 103 690
US - A - 4 161 952
US - A - 4 419 819

(73) Patentinhaber: Osypka, Peter, Dr. Ing., Basler Strasse 109, D-7889 Grenzach-Wyhlen (DE)

(72) Erfinder: Osypka, Peter, Dr., Hornrain 31, D-7889 Grenzach-Wyhlen (DE)
Erfinder: Höge, Rüdiger, Dr. med., Am Kasimir 12, D-6300 Giessen 8 (DE)

(74) Vertreter: Patentanwälte Dipl.-Ing. Hans Schmitt Dipl.-Ing. Wolfgang Maucher, Dreikönigstrasse 13, D-7800 Freiburg i.Br. (DE)

ACTORUM AG

**Beschreibung**

Die Erfindung betrifft eine chirurgische Elektrode zur zeitweiligen Überwachung oder Stimulation der Herztätigkeit, mit einer vorzugsweise gebogenen chirurgischen Nadel am in der Herzwand zu plazierenden und zu verankernden Ende der Elektrode, welche Nadel nach dem Einsetzen der Elektrode abgetrennt wird, und mit einem in Gebrauchsstellung durch den Thorax nach aussen ragenden Ende zum Verbinden mit einem äusseren Herzschrittmacher oder EKG-Monitor.

Eine derartige Elektrode, auch Herzdraht genannt, ist aus der US-A-4 010 756 bekannt. Sie besteht in der Regel aus feinen zu einer Elektrode verarbeiteten Edelstahldrähten, die mit einer körperverträglichen Kunststoffumhüllung versehen sind. Beide Enden dieser Elektrode sind auf eine Länge von ca. 2 cm nicht isoliert. Die zunächst mit dem einen Ende verbundene, in der Regel gebogene chirurgische Nadel wird während der Herzoperation in das Herzgewebe eingestochen, um den nicht isolierten Teil des Herzdrahtes, der gleichzeitig als Elektrode dient, in das Myocard des Herzens zu plazieren. Weiterhin ist eine derartige Elektrode aus der EP-A1-0 083 674 bekannt, wobei das in der Herzwand zu plazierende und zu verankernde Ende als hülsenartiger Pol ausgebildet ist, von welchem der die chirurgische Nadel aufweisende Zugfaden ausgeht. Dieser Faden ist nahe dem hülsenförmigen Pol als Wendel ausgebildet, welche nach dem Abtrennen der chirurgischen Nadel die Verankerung in der Herzwand untersützt.

Nach Fixierung der Elektrode wird also die Nadel abgeschnitten. Der andere Teil der Elektrode bzw. des Herzdrahtes ist zunächst mit einer geraden chirurgischen Nadel verbunden, die durch den Thorax gestochen wird und die Verbindung zu einem äusseren Herzschrittmacher oder EKG-Monitor herstellt. Bei auftretenden Rhythmusstörungen am Herzen kann so auf einfache Weise das Herz stimuliert werden. Nach etwa zwei bis drei Wochen werden diese Herzdrähte von aussen aus dem Herzen und dem Thorax herausgezogen.

Nachteilig ist bei diesen Elektroden, dass zur Stimulation jeweils wenigstens zwei solcher Elektroden notwendig sind, um zwei entsprechende elektrische Pole am Herzen unterzubringen. Dies erfordert zwei Einstiche in die Herzwand. Dabei ist eine entsprechend grössere Gefahr gegeben, dass ein solcher Elektrodendraht unabsichtlich aus dem Herzen herausgezogen werden kann. Dies muss mit entsprechenden chirurgischen Massnahmen verhindert werden. So wird beispielsweise das distale Ende einer solchen Elektrode etwas abgeknickt oder es wird eine leichte Ligatur um diese Elektrode gelegt. Gegebenenfalls wird auch eine Art Schlinge zur Verankerung am Herzen gebildet. All diese Massnahmen bedeuten einen zusätzlichen Aufwand und oft weitere Einstiche im Herzgewebe.

Der Erfindung liegt vor allem die Aufgabe zugrunde, eine Herz-Elektrode der eingangs erwähnten Art zu schaffen, bei der die Zahl der notwendigen Einstiche in das Muskelgewebe des Herzens vermindert und dennoch eine sichere und zuverlässige Stimulation bzw. Überwachungsfunktion möglich ist.

Die Lösung dieser Aufgabe besteht darin, dass die Elektrode im am Herzen zu fixierenden Bereich zwei mit Abstand zueinander angeordnete Pole aufweist, wobei zwei gegeneinander isolierte Drähte oder Leiter zu einem ersten Pol geführt sind, wo der eine der beiden elektrischen Leiter mündet, dass der zweite elektrische Leiter diesen ersten Pol isoliert durchläuft und mit Abstand zu dem ersten Pol den zweiten Pol und dahinter die abtrennbare chirurgische Nadel aufweist und dass der der Nadel nahe Pol als gegenüber dem Verlauf der Elektrode vorspringender, für den Eintritt in die Herzwand nachgiebiger Anker ausgebildet ist.

Dadurch ist es nun möglich, mit einer einzigen Elektrode der eingangs erwähnten Art eine Überwachung und Stimulation der Herztätigkeit zu machen, so dass nur ein einziger Einstich am Herzen notwendig ist. Es können gleichzeitig beide Pole mit Abstand zueinander in der Herzwand verankert werden. Somit ist nur ein einziger Einstich sowohl am Herzen als auch am Thorax erforderlich, was gleichzeitig auch das Anlegen der Elektroden beschleunigt. Die Verwendung zweier Pole, die beide in die Herzwand gelegt werden, verbessert ausserdem von vorneherein die Verankerung dieser Elektrode im Gewebe.

Der als Anker dienende Pol kann durch Aufspleissung des zu ihm führenden, aus Einzeldrähten z.B. litzenförmig zusammengesetzten elektrischen Leiters gebildet sein. Eine solche Aufspleissung des elektrischen Leiters an dessen Pol ist ohne weiteres vertrebar, da die Elektrode nur einmal verwendet wird. Eine solche Aufspleissung vermeidet dabei zusätzliche an der Elektrode anbringbare Ankerteile, wie sie insbesondere bei transvenös in das Herz einführbaren Elektroden häufig vorgesehen sind. Darüber hinaus wird durch eine solche Aufspleissung auch die Oberfläche vergrössert, was die Reizübertragung und somit die Stimulation des Herzens verbessert.

Es ist auch statt einer Aufspleissung oder zusätzlich dazu möglich, dass der Anker-Pol mäanderförmig, wellenförmig oder schraubenförmig gebogen ist. Eine entsprechend verbesserte Verankerung und Stimulation ist möglich. Beispielsweise kann zur Bildung des Anker-Poles die zu ihm führende Litze aufgespleisst sein und die dadurch bereichsweise voneinander getrennten Einzeldrähte oder Einzeldrahtgruppen können nun jeweils mäanderförmig, wellenförmig oder schraubenförmig ausgebildet sein. Es ergibt sich dann ein mehradriger Pol mit entsprechenden Verformungen, um die Verankerung am Herzen zu verbessern.

Der zurückliegende erste Pol der erfindungsgemässen bipolaren Elektrode kann als die in seinem Bereich zusammengeführten Leiter umschliessende, elektrisch leitende Klemmhülse ausgebildet sein, in deren Bereich der an diesem Pol mündende und endende elektrische Leiter abisoliert und vorzugsweise an der Aussenseite des weiterführenden Leiters befestigt, insbesondere um diesen gewickelt und mit der Innenseite der Hülse elektrisch leitend verbunden ist. Dabei ist es für das Einführen auch des zweiten Poles in die Herzwand zweckmässig, wenn das dem zweiten Pol zugewandte stirnseitige Ende der Klemmhülse konisch ausgebildet ist und wenn der zu dem zweiten Pol führende Leiter aus der dabei gebildeten Spitze der Klemmhülse austritt. Somit kann in einem Zuge durch entsprechendes Einführen zunächst der gebogenen chirurgischen Nadel der erste und nachfolgend gleichzeitig der zweite Pol der erfindungsgemässen bipolaren Elektrode in der Herzwand verankert werden. Die Anschlussdrähte können dann in üblicher Weise durch den Thorax nach aussen geführt werden. Dabei ist es vorteilhaft, wenn die Anschlussdrähte oder -leiter voneinander unterscheidbar sind. Dadurch können sie von aussen exakt bestimmt und in der richtigen Weise an einen Herzschrittmacher oder EKG-Monitor angeschlossen werden. Die Unterscheidbarkeit kann z.B. durch verschiedenfarbige Isolierungen od.dgl. erreicht werden.

Vor allem bei Kombination einzelner oder mehrerer der vorbeschriebenen Merkmale und Massnahmen ergibt sich eine bipolare chirurgische Elektrode, die nur einen Einstich in das Myocardgewebe erforderlich macht. Dabei entsteht ein definierter Abstand zwischen den beiden Elektroden bzw. den Polen und damit die Vermeidung von Kurzschlüssen, die dann auftreten könnten, wenn sowohl im Vorhof als auch im Ventrikel zwei einzelne Herzdrähte der eingangs beschriebenen Art plaziert werden müssen. Ferner ergibt sich insbesondere bei Ausbildung eines Anker-Poles eine bessere Verankerung und durch die damit einhergehende Vergrösserung der Oberfläche der proximalen Elektrode eine bessere Stimulation und Reizübertragung bzw. eine Verringerung der Reizschwelle bei der Stimulation, was ein ganz wesentlicher Vorteil ist.

Nachstehend ist die Erfindung mit ihren ihr als wesentlich zugehörenden Einzelheiten anhand der Zeichnung noch näher beschrieben.

Es zeigt in zum Teil schematisierter Darstellung:

Fig. 1 die Anordnung einer erfindungsgemässen Elektrode in bipolarer Ausführung am Herzen,

Fig. 2 ein Ausführungsbeispiel einer bipolaren Elektrode, deren zweiter Pol zur Verbesserung seiner Verankerung aufgespleisst ist, wobei die chirurgische Nadel zum Anlegen der Elektrode noch vorhanden ist,

Fig. 3 bis 9 der Fig. 2 entsprechende Darstellungen, jeweils ohne die chirurgische Nadel, wobei der Verankerungs-Pol jeweils unterschiedlich ausgebildet ist, sowie

Fig. 10 in vergrösserter Darstellung einen Längsschnitt durch den ersten Pol.

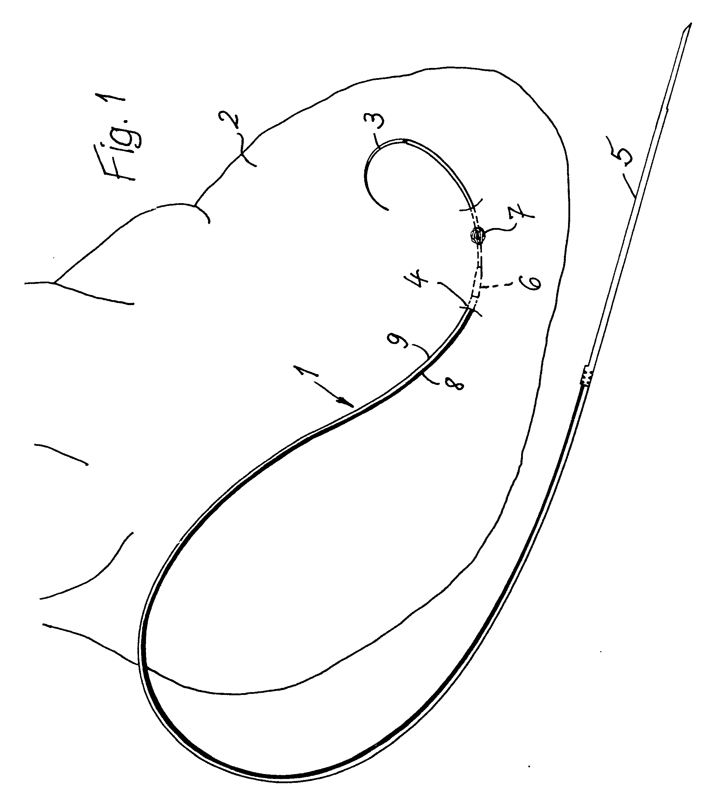

Eine im ganzen mit 1 bezeichnete chirurgische Elektrode dient zur zeitweiligen Überwachung und/oder Stimulation der Tätigkeit eines Herzens 2 und hat für ihre Befestigung am Herzen eine gebogene chirurgische Nadel 3, mit der an einer entsprechenden Stelle 4 der Herzwand eingestochen werden kann, um die Elektrode 1 durch diesen Einstich hindurchziehen zu können. Nach dem in Fig. 1 angedeuteten Einsetzen der Elektrode 1 kann die chirurgische Nadel 2 abgetrennt werden. Zu der Elektrode 1 gehört ferner ein nach aussen durch den Thorax zu führendes Ende 5, welche zum Anschliessen eines äusseren Herzschrittmachers oder EKG-Monitors dient.

In allen Ausführungsbeispielen ist vorgesehen, dass die Elektrode 1 im am Herzen 2 zu fixierenden Bereich zwei mit Abstand zueinander angeordnete Pole 6 und 7 aufweist, wobei zwei gegeneinander isolierte Drähte oder Leiter 8 und 9 zu dem ersten Pol 6 geführt sind, wo der eine elektrische Leiter 8 mündet. Der zweite elektrische Leiter 9 durchläuft den ersten Pol 6 isoliert und weist mit Abstand zu dem ersten Pol 6 den zweiten Pol 7 und dahinter die abtrennbare chirurgische Nadel 3 auf. Diese Anordnung wird besonders deutlich anhand der Fig. 1, 2 und 10.

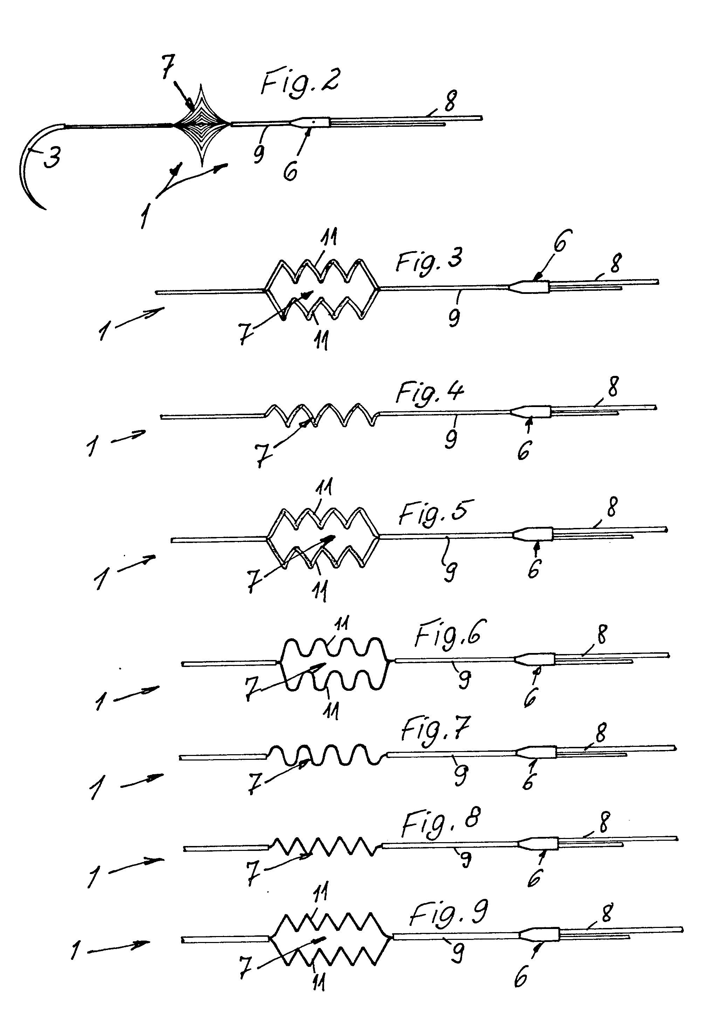

In den Fig. 2 bis 10 ist dargestellt, dass der der gebogenen Nadel 3 nahe Pol 7 als gegenüber dem Verlauf der Elektrode 1 vorspringender, für den Eintritt in die Herzwand nachgiebiger Anker ausgebildet ist. Dabei sind verschiedene Ausführungsformen möglich.

Gemäss Fig. 2 kann der als Anker dienende Pol 7 durch Aufspleissen des zu ihm führenden aus Einzeldrähten z.B. litzenförmig zusammengesetzten elektrischen Leiters 9 gebildet sein. Es entsteht dann bei entsprechender Formgebung der aufgespleissten Einzeldrähte 10 ein federnd nachgiebiger, abisolierter Bereich, der als nachgiebiger Pol 7 gut in der Herzwand verankert werden kann, dort aber auch eine bessere Verankerung und zusätzlich eine bessere Reizleitung bewirkt.

Die Fig. 4, 7 und 8 zeigen Ausführungsbeispiele, bei denen der Anker-Pol 7 mäanderförmig, wellenförmig oder schraubenförmig gebogen ist, so dass dieser wiederum abisolierte Bereich eine Oberflächenvergrösserung und Verbesserung der Reizübertragung bei gleichzeitig verbesserter Verankerung bewirkt.

Die Fig. 3, 5, 6, 9 und 10 sind Beispiele, bei denen zur Bildung des Verankerungspoles 7 der zu ihm führende Leiter aufgespleisst und die dadurch bereichsweise voneinander getrennten Einzeldrähte 11 oder Einzeldraht-Gruppen mäanderförmig, wellenförmig oder schraubenförmig ausgebildet sind. Dabei können die schraubenförmigen Einzeldrähte 11 entweder im gleichen Sinne oder im entgegengesetzten Sinne gewunden sein, worin der Unterschied zwischen den Fig. 3 und 5 besteht. Diese vereinzelten Litzen oder Drähte des Leiters 9 im Bereich des Poles 7 können durch das Einziehen in die von der Nadel 3 gebildeten Öffnung nachgeben, wobei sie aber eine Verformung erfahren. Die Rückstellkräfte dieser Verformung verbessern die Verankerung und den Kontakt mit dem Herzgewebe.

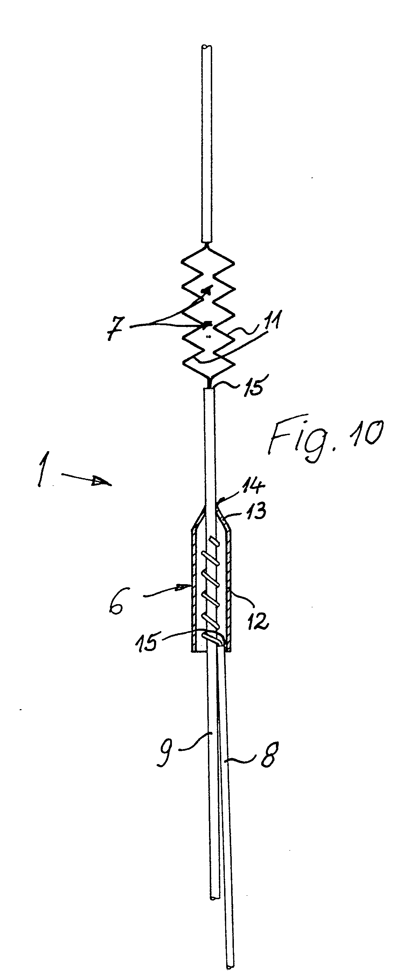

In Fig. 10 erkennt man, dass der gegenüber dem Pol 7 zurückliegende, ebenfalls in der Herzwand unterzubringende erste Pol 6 als die in seinem Bereich zusammengeführten Leiter 8 und 9 umschliessende, elektrisch leitende Klemmhülse 12 ausgebildet ist, in deren Bereich der an diesem Pol 6 mündende elektrische Leiter 8 abisoliert und im Ausführungsbeispiel an der Aussenseite des weiterführenden Leiters 9 befestigt und zwar um diesen gewickelt ist. Er wird mit der Innenseite der Hülse 12 elektrisch leitend verbunden. Das dem zweiten Pol 7 zugewandte stirnseitige Ende 13 der Klemmhülse 12 ist dabei konisch angespitzt und der zu dem zweiten Pol 7 führende Leiter 9 tritt aus der dabei gebildeten Spitze 15 aus. Somit bildet dieser Leiter 9 bei seinem Einziehen in das Herzgewebe eine Führung, der dann die Spitze 14, der konische Bereich 13 und schliesslich die eigentliche Klemmhülse 12 folgen können. Dadurch ist auch die Verankerung dieses Poles 6 in einem Zuge mit der Befestigung der gesamten Elektrode 1 möglich.

In Fig. 10 ist noch angedeutet, dass die Anschlussdrähte oder Leiter 8 und 9 voneinander unterscheidbar sind, wobei sie im Ausführungsbeispiel unterschiedliche Aussenmasse haben. Diese Unterscheidbarkeit könnte aber zusätzlich oder stattdessen auch durch unterchiedliche Farben der Isolierung 15 erreicht werden.

Insgesamt ergibt sich ein einfacher und preiswerter Aufbau einer Elektrode 1, die zur zeitweiligen Überwachung und/oder Stimulation des Herzens 2 an diesem in üblicher Weise angelegt werden kann, wodurch durch einen Einstich in die Wand des Herzens 2 gleichzeitig zwei Pole 6 und 7 verankert werden können, die somit auch einen gleichbleibenden festen Abstand zueinander behalten, so dass kein Kurzschluss durch Bewegungen des Herzens oder des Patienten entstehen kann. Dennoch bleibt die Art der zum Verlegen dieser Elektrode 1 durchführbaren Operation unverändert. Die Zahl der Einstiche am Herzen wird aber verringert.

Alle in der Beschreibung, den Ansprüchen, der Zusammenfassung und der Zeichnung dargestellten Merkmale und Konstruktionsdetails können sowohl einzeln als auch in beliebiger Kombination miteinander wesentliche Bedeutung haben.

**Patentansprüche**

1. Chirurgische Elektrode (1) zur zeitweiligen Überwachung oder Stimulation der Herztätigkeit, mit einer vorzugsweise gebogenen chirurgischen Nadel (3) am in der Herzwand zu plazierenden und zu verankernden Ende der Elektrode, welche Nadel (3) nach dem Einsetzen der Elektrode (1) abgetrennt wird, und mit einem in Gebrauchsstellung durch den Thorax nach aussen ragenden Ende (5) zum Verbinden mit einem äusseren Herzschrittmacher oder EKG-Monitor, dadurch gekennzeichnet, dass die Elektrode (1) im am Herzen (2) zu fixierenden Bereich zwei mit Abstand zueinander angeordnete Pole (6 und 7) aufweist, wobei zwei gegeneinander isolierte Drähte oder Leiter (8 und 9) zu einem ersten Pol (6) geführt sind, wo der eine elektrische Leiter (8) mündet, dass der zweite elektrische Leiter (9) diesen ersten Pol (6) isoliert durchläuft und mit Abstand zu dem ersten Pol (6) den zweiten Pol (7) und dahinter die abtrennbare chirurgische Nadel (3) aufweist und dass der der Nadel (3) nähere Pol (7) als gegenüber dem Verlauf der Elektrode (1) vorspringender, für den Eintritt in die Herzwand nachgiebiger Anker ausgebildet ist.

2. Elektrode nach Anspruch 1, dadurch gekennzeichnet, dass der als Anker dienende Pol (7) durch Aufspleissung des zu ihm führenden, aus Einzeldrähten z. B. litzenförmig zusammengesetzten elektrischen Leiters (9) gebildet ist.

3. Elektrode nach Anspruch 1, dadurch gekennzeichnet, dass der Anker-Pol (7) mäanderförmig, wellenförmig oder schraubenförmig gebogen ist.

4. Elektrode nach einem der vorstehenden Ansprüche, dadurch gekennzeichnet, dass zur Bildung des Anker-Poles (7) der zu ihm führende Leiter aufgespleisst ist und die dadurch bereichsweise voneinander getrennten Einzeldrähte (11) oder Einzeldraht-Gruppen mäanderförmig, wellenförmig oder schraubenförmig ausgebildet sind.

5. Elektrode nach einem der Ansprüche 1 bis 4, dadurch gekennzeichnet, dass der zurückliegende erste Pol (6) als die in seinem Bereich zusammengeführten Leiter (8 und 9) umschliessende, elektrisch leitende Klemmhülse (12) ausgebildet ist, in deren Bereich der an diesem Pol (6) mündende elektrische Leiter (8) abisoliert und vorzugsweise an der Aussenseite des weiterführenden Leiters (9) befestigt, insbesondere um diesen gewickelt und mit der Innenseite der Hülse (12) elektrisch leitend verbunden ist.

6. Elektrode nach einem der Ansprüche 1 bis 5, dadurch gekennzeichnet, dass das dem zweiten Pol (7) zugewandte stirnseitige Ende (13) der Klemmhülse (12) konisch ausgebildet ist und dass der zu dem zweiten Pol (7) führende Leiter (9) aus der dabei gebildeten Spitze (14) der Klemmhülse (12) austritt.

7. Elektrode nach einem der Ansprüche 1 bis 6, dadurch gekennzeichnet, dass die Anschlussdrähte oder -leiter voneinander unterscheidbar sind.

**Claims**

1. A surgical electrode (1) for temporarily monitoring or stimulating cardiac activity, including at the electrode end to be placed and anchored in the cardiac wall a surgical needle (3), preferably a bent one, said needle (3) being detached after insertion of the electrode (1), and including an end (5) which in the position for use projects outwardly through the thorax and serves for connection with an external cardiac pacemaker or electrocardiogram monitor, characterized in that in the area to be fixed to the heart (2) the electrode (1) has two poles (6 and 7) disposed in spaced relationship to one another, two wires or leads (8 and 9) insulated with respect to one another being guided to a first pole (6) where the one electric lead (8) ends, that the second electric lead (9) traverses said first pole (6) in an insulated condition and has the second pole (7) in spaced relationship to the first pole (6) and behind that the detachable surgical needle (3) and that the pole (7) closer to the needle (3) takes the form of an anchor which protrudes relative to the path of the electrode (1) and is yielding for entry into the cardiac wall.

2. The electrode as claimed in claim 1, characterized in that the pole (7) serving as an anchor is formed by splicing the electric lead (9) which leads to said pole and is composed of single wires put together in e.g. a strand-like manner.

3. The electrode as claimed in claim 1, characterized in that the anchor pole (7) is bent in a meander-like, wave-like or spiral manner.

4. The electrode as claimed in any one of the preceding claims, characterized in that to form the anchor pole (7) the lead leading thereto is spliced and the single wires (11) or groups thereof thereby separated from one another in areas are devised to be meander-like, wave-like or spiral.

5. The electrode as claimed in any one of claims 1 to 4, characterized in that the rearward first pole (6) takes the form of the electrically conductive clamping sleeve (12) which encloses the leads (8 and 9) brought together in its region, the electric lead (8) ending at said pole (6) being bared in the region of the clamping sleeve and preferably being fastened to the outside of the continuing lead (9), in particular being wound around the latter and being connected in an electrically conductive manner to the inside of the sleeve (12).

6. The electrode as claimed in any one of claims 1 to 5, characterized in that the front end (13) which belongs to the clamping sleeve (12) and faces the second pole (7) is devised to be conical and that the lead (9) leading to the second pole (7) departs from the thereby formed tip (14) of the clamping sleeve (12).

7. The electrode as claimed in any one of claims 1 to 6, characterized in that the connecting wires or leads are distinguishable from one another.

**Revendications**

1. Electrode chirurgicale (1) pour la surveillance ou la stimulation temporelle de l'activité cardiaque, comprenant une aiguille chirurgicale (3) de préférence cintrée à l'extrémité de l'électrode devant être placée ou ancrée dans la cloison cardiaque, ladite aiguille (3) étant séparée après l'engagement de l'électrode (1), ainsi qu'une extrémité (5) qui dépasse vers l'extérieur à travers le thorax, en position d'utilisation, et est destinée à être raccordée à un stimulateur cardiaque ou à un écran de contrôle d'ECG extérieur, caractérisée par le fait que l'électrode (1) présente, dans la région devant être assujettie au cœur (2), deux

pôles (6 et 7) disposés à distance l'un de l'autre, deux fils ou conducteurs (8 et 9) mutuellement isolés gagnant un premier pôle (6) où débouche l'un (8) des conducteurs électriques; par le fait que le second conducteur électrique (9) franchit ce premier pôle (6) avec isolation et présente le second pôle (7) à distance du premier pôle (6), puis l'aiguille chirurgicale séparable (3) derrière ledit second pôle; et par le fait que le pôle (7) le plus rapproché de l'aiguille (3) est réalisé en tant qu'induit qui fait saillie par rapport à l'étendue de l'électrode (1), et est doué de souplesse en vue de pénétrer dans la cloison cardiaque.

2. Electrode selon la revendication 1, caractérisée par le fait que le pôle (7) servant d'induit est formé par épissure ouverte du conducteur électrique (9) qui le rejoint et est, par exemple, structuré en forme de cordon tressé à partir de fils individuels.

3. Electrode selon la revendication 1, caractérisée par le fait que le pôle-induit (7) est coudé en forme de méandres, d'ondulations ou d'hélice.

4. Electrode selon l'une des revendications précédentes, caractérisée par le fait que, pour former le pôle-induit (7), le conducteur qui le rejoint est ouvert par épissage et les fils individuels (11) ou groupes de fils individuels, qui sont de la sorte séparés par zones les uns des autres, sont réalisés en forme de méandres, d'ondulations ou d'hélice.

5. Electrode selon l'une des revendications 1 à 4, caractérisée par le fait que le premier pôle (6) en retrait est réalisé sous la forme d'une douille de serrage (12) électriquement conductrice qui entoure les conducteurs (8 et 9) regroupés dans sa région, et au voisinage de laquelle le conducteur électrique (8) qui s'achève à ce pôle (6) est isolé et est fixé, de préférence, à la face externe du conducteur (9) poursuivant son cheminement, en étant notamment enroulé autour de ce dernier, et est relié avec conduction électrique à la face interne de la douille (12).

6. Electrode selon l'une des revendications 1 à 5, caractérisée par le fait que l'extrémité frontale (13) de la douille de serrage (12), tournée vers le second pôle (7), est de réalisation conique; et par le fait que le conducteur (9) qui gagne le second pôle (7) sort de la pointe (14) de la douille de serrage (12), à laquelle il est ainsi donné naissance.

7. Electrode selon l'une des revendications 1 à 6, caractérisée par le fait que les fils ou conducteurs de raccordement peuvent être distingués les uns des autres.

Fig. 1

Fig.2

Fig.3

Fig.4

Fig.5

Fig.6

Fig.7

Fig.8

Fig.9

Fig. 10